# EUROPEAN PATENT SPECIFICATION

(11) **EP 2 040 647 B1**
(45) Date of publication and mention of the grant of the patent: **29.10.2014**
(21) Application number: 07789445.9
(22) Date of filing: 25.06.2007
(51) Int. Cl.: A61F 2/42

(54) **ARTICULATION PROSTHESIS FOR SMALL BONES, IN PARTICULAR FOR PHALANGEAL, METACARPO-PHALANGEAL OR METATARSO-PHALANGEAL ARTICULATIONS**
ARTIKULATIONSPROTHESE FÜR KLEINE KNOCHEN, INSBESONDERE FÜR PHALANGEAL-, METACARPOPHALANGEAL- ODER METATARSOPHALANGEAL-GELENKE
PROTHÈSE D'ARTICULATION POUR DES PETITS OS, EN PARTICULIER POUR LES ARTICULATIONS DES PHALANGES, DES MÉTACARPES OU DES MÉTATARSES

(30) Priority: 23.06.2006 US 815867 P; 18.07.2006 FR 0606503
(43) Date of publication of application: 01.04.2009
(73) Proprietor: Implants International Limited, Cleveland Thornaby-on-Tees TS17 9LZ (GB)
(72) Inventor: EMMANUEL, Mohan, Station Town Durham TS28 5NA (GB)
(74) Representative: Jeannet, Olivier
(86) International application number: PCT/IB2007/001735
(87) International publication number: WO 2007/148220

(56) References cited:
- EP-A- 1 508 316
- WO-A-96/37169
- WO-A1-2005/009298
- FR-A- 2 706 287
- FR-A1- 2 733 412

## Description

The present invention relates to a joint prosthesis for small bones, particularly for phalangeal, metacarpo-phalangeal or metatarso-phalangeal joints.

A joint prosthesis for small bones is known, comprising a first anchoring element in one of the bones, forming a joint surface, a second anchoring element in the other bone and a meniscal element able to be installed on this second anchoring element.

The dimensions of the bone anchoring elements were reduced. The problem exists for this type of prosthesis of obtaining reliable installation of the meniscal element on the corresponding bone anchoring element, and to be able to carry out this assembly easily and quickly.

The existing prostheses are not completely satisfactory from this point of view, and the primary objective of the invention is to resolve this drawback.

Moreover, existing prostheses do not always make it possible to easily place the meniscal element in a set position relative to the bone anchoring element intended to receive it.

The invention also has the objective of proposing a prosthesis resolving this drawback.

The document Nr. WO 2005/009298 discloses the features cited in the preamble of claim 1.

The invention is as defined by the characterizing part of claim 1.

The meniscal element of the prosthesis according to the invention thus comprises a slotted assembly shaft, defining two parallel distal portions, and a receiving hole with locking of the corresponding transverse rod of the bone anchoring element. In practice, upon insertion of the assembly shaft in the cavity of the bone anchoring element, said parallel distal portions encounter the transverse rod, are elastically distanced from each other by insertion of this transverse rod between them and then, when the transverse rod penetrates said transverse hole, resume their initial position, ensuring retention of the transverse rod in the transverse hole and, because of this, assembly of the meniscal element in this set position.

When the meniscal element must be placed in a set position relative to the anchoring element, in particular when it comprises a rib or groove for guiding the movement of said first anchoring element, said transverse rod and said slot make it possible to place the meniscal element in this set position easily.

If necessary, the assembly shaft of the meniscal element comprises one or several recesses or flats at the level of at least one of the openings through which said transverse hole opens to the outside of this assembly shaft.

This or these recess(es) or flat(s) make it possible to reduce the cross-section of the assembly shaft in this place in order to increase the flexibility of said parallel distal portions defined by the slot.

Said medullary anchoring shaft of said second bone anchoring element and said assembly shaft of the meniscal element may have a tapered shape.

Said second anchoring element may have a collar for receiving the meniscal element. This collar may be connected to said medullary anchoring shaft by a flared portion, and said transverse rod may be located immediately below this flared portion.

The invention shall be clearly understood, and other characteristics and advantages thereof shall appear, in reference to the annexed diagrammatic drawing, illustrating, as a non-exhaustive example, a preferred embodiment of a bone anchoring element and of a meniscal element comprised by the prosthesis in question.
Figure 1 is a perspective view of this bone anchoring element and the meniscal element assembled on said bone anchoring element;
Figure 2 is a cross-section of these elements following the axis of these elements;
Figures 3 to 5 are views of the meniscal elements from a side according to a first direction, a side according to a second direction, perpendicular to this first direction, and from below, respectively, and
Figures 6 to 8 are views of the bone anchoring element from a side according to a first direction, a side according to a second direction, perpendicular to this first direction, and from below, respectively.

The figures illustrate an anchoring element 1 and a meniscal element 2 which are part of a joint prosthesis for small bones, particularly for phalangeal, metacarpo-phalangeal and metatarso-phalangeal joints.

The anchoring element 1 is intended to be anchored in one of the bones forming the joint to be treated, and the prosthesis also comprises another bone anchoring element (not shown) intended to be anchored in the other bone forming the joint to be treated. This other bone anchoring element comprises a joint surface intended to cooperate with a joint surface formed by the meniscal element 2.

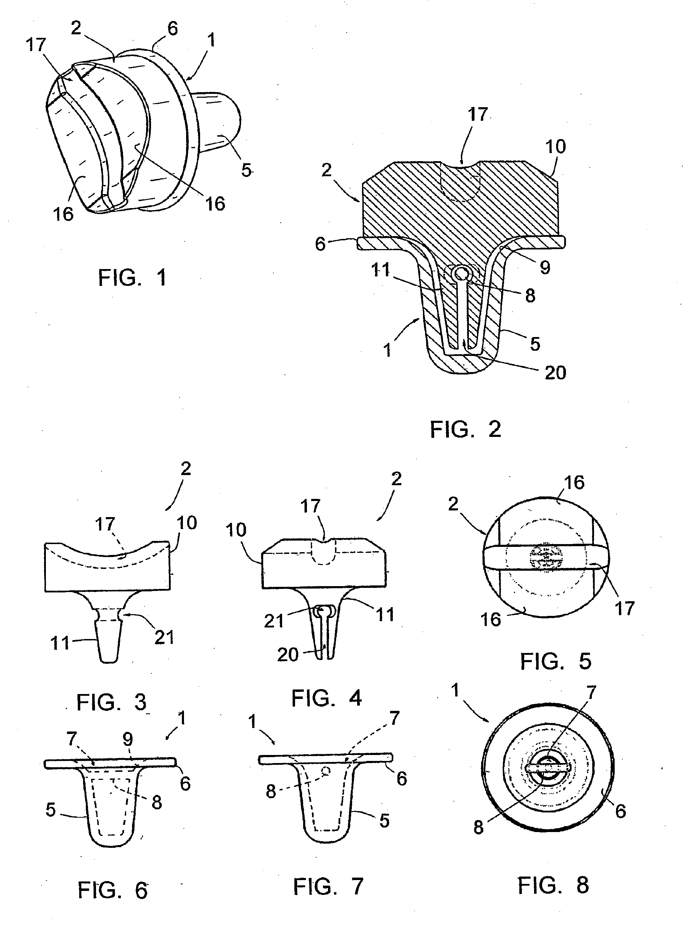

As shown in figures 2 and 6 to 8, the anchoring element 1 comprises a medullary anchoring shaft 5 and a collar 6. The assembly is formed by a single piece of an appropriate metallic material, particularly in a chrome-cobalt alloy.

The medullary anchor shaft 5 is tapered in shape and is hollow inside, defining a cavity 7, and comprises a transverse rod 8 fixed to it, extending through this cavity 7. This medullary anchor shaft 5 is connected to the collar 6 by a flared portion 9 and the transverse rod 8 is located immediately below this flared portion 9.

The meniscal element 2 comprises a part 10 in the shape of a plateau and an assembly shaft 11. The assembly is formed by a piece of a material promoting sliding, for example in high-density polyethylene.

The part 10 forms said joint surface; in the illustrated example, this joint surface presents lateral glenoid surfaces 16 and a median guide groove 17, intended to cooperate with lateral condyle surfaces and with a median rib comprised by the joint surface of said other bone anchoring element, respectively. The part 10 also forms, from the side of the assembly shaft 11, a flat surface intended to be received on the collar 6.

The assembly shaft 11 is tapered in shape and connected to the part 10 by a flared area. It presents one longitudinal slot 20 having a width smaller than that of the transverse section of the transverse rod 8, one end of which opens in the free end of the assembly shaft 11 and the other end of which opens in a transverse hole 21 having a cross-section slightly larger than that of said transverse rod 8. The assembly shaft 11 also comprises two flats at the level of at least one of the openings through which the hole 21 opens to the outside of this assembly shaft.

As can be seen in reference to figure 2, the meniscal element 2 is intended to be assembled on the anchoring element 1 by engagement of the assembly shaft 11 in the cavity 7 of the anchor shaft 5. The assembly shaft 11 is intended, thanks to the slot 20, to be engaged on the transverse rod 8 until engagement of this rod 8 in the hole 21, with locking, when the meniscal element 2 is in the assembled position on the anchor element 1. In practice, during insertion of the assembly shaft 11 in the cavity 7, the parallel distal portions of this shaft 11 defined by the slot 20 come into contact with the transverse rod 8, are elastically distanced from each other by insertion of this rod 8 between them then, when the rod 8 penetrates the hole 21, resume their initial position, ensuring retention of the rod 8 in the hole 21 and, because of this, assembly of the meniscal element 2 on the anchor element 1.

As shown by the preceding, the invention provides a joint prosthesis presenting determining advantages to enable reliable assembly of the meniscal element on the bone anchoring element, able to be performed easily and quickly, and to allow easy placement of the meniscal element in a set position relative to the anchoring element.

It goes without saying that the invention is not limited to the embodiment described above as an example, but that it extends to all embodiments covered by the annexed claims.

## Claims

1. Joint prosthesis, comprising a first anchoring element capable of being installed in one of the bones, forming a joint surface, a second anchoring element (1) capable of being installed in the other bone and a meniscal element (2) capable of being installed on this second anchoring element (1);
- said second bone anchoring element (1) comprises a medullary anchor shaft (5) with a hollow interior, defining a cavity (7), and a transverse rod (8) fixed such that it extends through this cavity (7), and
- the meniscal element (2) comprises an assembly shaft (11) suitable to be engaged in said cavity (7), this assembly shaft (11) having a longitudinal slot (20) having a first end which opens in the free end of the assembly shaft (11), said this assembly shaft (11) being capable to be engaged on said transverse rod (8);
**characterized in that**:
- said joint prosthesis is a phalangeal, metacarpo-phalangeal or metatarso-phalangeal joint;
- said slot (20) has a width smaller than the transverse section of said transverse rod (8) and has a second end which opens in a transverse hole (21) having a cross-section slightly larger than that of said transverse rod (8), said assembly shaft (11) being suitable, thanks to this slot (20), to be engaged on said transverse rod (8) until engagement of said transverse rod (8) in said transverse hole (21), with locking, when the meniscal element (2) is in its assembled position on said second bone anchoring element (1).

2. Prosthesis according to claim 1, **characterized in that** the meniscal element (2) comprises a rib or groove (17) for guiding the movement of said first anchoring element (1)

3. Prosthesis according to claim 1 or claim 2, **characterized in that** the assembly shaft (11) of the meniscal element (2) comprises one or several recesses or flats at the level of at least one of the openings through which said transverse hole (21) opens to the outside of this assembly shaft (11).

4. Prosthesis according to any of claims 1 to 3, **characterized in that** said medullary anchoring shaft (5) of said second bone anchoring element (1) and said assembly shaft (11) of the meniscal element (2) have a tapered shape.

5. Prosthesis according to any of claims 1 to 4, **characterized in that** said second anchoring element (1) has a collar (6) for receiving the meniscal element (2).

6. Prosthesis according to claim 5, **characterized in that** said collar (6) is connected to said medullary anchoring shaft (5) by a flared portion (9), and **in that** said transverse rod (8) is located immediately below this flared portion (9).

## Patentansprüche

1. Gelenkprothese, mit einem ersten Verankerungselement in einem Knochen Bildung einer Gelenkfläche befestigt ist, und einem Meniskuselement (2) auf dem zweiten Verankerungselement (1) kann montiert ist;
- der zweiten Verankerungselement (1) mit einem medullären Verankerung Schwanz (5) mit einem hohlen Innenraum, die einen Hohlraum definiert (7), und eine transversal stab (8) daran befestigt ist, der sich durch den Hohlraum (7), und
- das Meniskuselement (2) mit einem Montageschaft (11), in dem Hohlraum (7) in Eingriff gebracht werden kann, Dieser Montageschaft (11) mit einem Längsschlitz (20) mit einem ersten Ende, dass in das freie Ende der Montagewelle öffnet (11), das in eröffnet das freie Ende des Schwanzanordnung (11), der Montageschaft (11) mit die transversal stab (8) kann Eingriff;
**dadurch gekennzeichnet, dass**:
- die Gelenkprothese ist eine phalangealen, Fingergrund oder metatarso Grundgelenk;
- der Schlitz (20) eine Breite hat, die kleiner als der Querschnitt die transversal stab (8) und ein zweites Ende, die in einer transversal loch (21) mündet, der Schlitz (20) eine Breite hat, die kleiner als der Querschnitt die transversal stab (8) und ein zweites Ende, die in einer transversal loch (21) mündet, der einen Querschnitt etwas größer als der die transversal stab (8), der Montageschaft (11) angepasst ist, durch den Schlitz (20) in Eingriff gebracht werden, um die transversal stab (8) bis zum Eingriff die transversal stab (8) in der transversal loch (21) mit Verriegelung, wenn der Meniskuselement (2) in Einbaulage auf dem zweiten Verankerungselement (1) Knochen ist.

2. Prothese nach Anspruch 1, **dadurch gekennzeichnet, dass** das Meniskuselement (2) eine Rippe oder eine Nut (17) zur Führung der Bewegung des ersten Verankerungselements (1) aufweist.

3. Prothese nach Anspruch 1 oder Anspruch 2, **dadurch gekennzeichnet, daß** der Montageschaft (11) des Meniskuselement (2) aufweist eine oder mehrere Ausnehmungen oder Wohnungen an mindestens einer der Öffnungen, durch die der transversal loch (21) zu der Außenseite der Montageschaft (11) öffnet sich.

4. Prothese nach einem der Ansprüche 1 bis 3, **dadurch gekennzeichnet, daß** der Befestigungsstegmark das zweite Verankerungselement (1) und der Montageschaft (11) des Meniskuselement (2) kegelstumpfförmig sind.

5. Prothese nach einem der Ansprüche 1 bis 4, **dadurch gekennzeichnet, dass** das zweite Verankerungselement (1) einen Kragen (6) zur Aufnahme das Meniskuselement (2) aufweist ist.

6. Prothese nach Anspruch 5, **dadurch gekennzeichnet, dass** die Aufnahme Kragen (6) mit der Rückenverankerungs Schwanz durch einen erweiterten Abschnitt (9) verbunden ist, und daß der Querstab (8) unmittelbar unterhalb des erweiterten Abschnitts (9) befindet ist.

## Revendications

1. Prothèse d'articulation, comprenant un premier élément d'ancrage pouvant être monté dans l'un des os, formant une surface d'articulation, un deuxième élément d'ancrage (1) pouvant être monté dans l'autre os et un élément méniscal (2) pouvant être monté sur ce deuxième élément d'ancrage (1) ;
- ledit deuxième élément d'ancrage (1) comprend une queue médullaire d'ancrage (5) creuse intérieurement, délimitant une cavité (7), et une tige transversale (8) fixée à lui s'étendant au travers de cette cavité (7), et
- l'élément méniscal (2) comprend une queue d'assemblage (11) propre à être engagée dans ladite cavité (7), cette queue d'assemblage (11) présentant une fente longitudinale (20) ayant une première extrémité qui débouche dans l'extrémité libre de la queue d'assemblage (11), ladite queue d'assemblage (11) pouvant être engagée sur ladite tige transversale (8) ;
**caractérisée en ce que** :
- ladite prothèse d'articulation concerne des articulations phalangiennes, métacarpo-phalangiennes ou métatarso-phalangiennes ;
- ladite fente (20) est de largeur inférieure à la section transversale de ladite tige transversale (8) et comprend une deuxième extrémité débouchant dans un trou transversal (21) de section légèrement supérieure à celle de ladite tige transversale (8), ladite queue d'assemblage (11) étant propre, grâce à cette fente (20), à être engagée sur ladite tige transversale (8) jusqu'à engagement de ladite tige transversale (8) dans ledit trou transversal (21), avec encliquetage, lorsque l'élément méniscal (2) est en position de montage sur ledit deuxième élément d'ancrage (1) osseux.

2. Prothèse selon la revendication 1, **caractérisée en ce que** l'élément méniscal (2) comprend une nervure ou une rainure (17) de guidage du mouvement dudit premier élément d'ancrage (1).

3. Prothèse selon la revendication 1 ou la revendication 2, **caractérisée en ce que** la queue d'assemblage (11) de l'élément méniscal (2) comprend un ou plusieurs évidements ou méplats au niveau d'au moins une des ouvertures par lesquelles ledit trou transversal (21) débouche sur l'extérieur de cette queue d'assemblage (11).

4. Prothèse selon l'une des revendications 1 à 3, **caractérisée en ce que** ladite queue médullaire d'ancrage dudit deuxième élément d'ancrage (1) osseux et ladite queue d'assemblage (11) de l'élément méniscal (2) sont de forme tronconique.

5. Prothèse selon l'une des revendications 1 à 4, **caractérisée en ce que** ledit deuxième élément d'ancrage (1) présente une collerette (6) de réception de l'élément méniscal (2).

6. Prothèse selon la revendication 5, **caractérisée en ce que** ladite collerette de réception (6) est raccordée à ladite queue médullaire d'ancrage par une portion évasée (9) et **en ce que** ladite tige transversale (8) est située immédiatement en dessous de cette portion évasée (9).
